(19) Europäisches Patentamt
European Patent Office
Office européen des brevets

(11) EP 1 889 858 B1

(12) # EUROPEAN PATENT SPECIFICATION

(45) Date of publication and mention of the grant of the patent:
**13.05.2009 Bulletin 2009/20**

(51) Int Cl.:
***C08F 293/00*** *(2006.01)* ***B01F 17/00*** *(2006.01)*

(21) Application number: **06291322.3**

(22) Date of filing: **17.08.2006**

(54) **Block copolymer, process for making the same, and use in emulsions**

Blockcopolymere, Verfahren zu deren Herstellung und ihre Verwendung in Emulsionen

Copolymère à blocs, procédé d'obtention et usage en émulsion

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**20.02.2008 Bulletin 2008/08**

(73) Proprietor: **Rhodia Opérations**
**93306 Aubervilliers (FR)**

(72) Inventors:
- **Deroo, Sophie**
  **92240 L'Hay les Roses (FR)**
- **Touzet, Sylvie**
  **92160 Antony (FR)**
- **Bzducha, Wojciech**
  **92400 Coubevoie (FR)**
- **Destarac, Mathias**
  **75005 Paris (FR)**

(74) Representative: **Boittiaux, Vincent et al**
**Rhodia Services Dir. Pro. Ind.**
**40 Rue de la Haie Coq.**
**93300 Aubervilliers (FR)**

(56) References cited:
**WO-A-20/04103327**
**US-A1- 2002 198 347**
**US-A1- 2006 088 487**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 889 858 B1

## Description

### BACKGROUND OF THE INVENTION

The present invention relates to new amphiphilic block copolymers and to use of block copolymers in emulsions, especially water in oil emulsions.

Amphiphilic block copolymers have been described in numerous documents. Document WO 03/068827 describes using some amphiphilic block in direct emulsions. Document WO 03/068848 describes using some block copolymers in water in oil emulsions, to control the stability of said emulsions.

Controlling the droplets size and/or the stability of emulsions (i.e. avoiding demixion coalescence, flocculation and/or creaming) is an issue to be addressed for many purposes. In consumer goods, there is a need for emulsions to have a long lifetime, as well for it to keep its properties as for it to keep a good aspect. Examples of consumer goods comprising simple water-in-oil emulsions are cosmetic compositions such as make-up, and skin-care creams, for example sunscreens, and more particularly waterproof sunscreens... Simple water-in-oil emulsions are also used in the field of explosive products. Stability is a particularly important property in that field. Other fields wherein simple water-in-oil emulsions are used include some anti-foaming compositions used in paper industry; water-in-oil emulsion polymerization, fracturing fluids used in oil fields, vehicles diesel gas (green diesel).

There is a need for new emulsifiers or emulsifier mixtures, that would provide an emulsification (droplets size) and/or a stability as good as and/or better than those of current emulsifiers or emulsifier mixtures, for some different phases or at different conditions, for example at higher temperatures.

More particularly, there is a need for different emulsions, and/or for improving the emulsions that have been described. There a need in particular for improving water in oil emulsions comprising a hydrocarbon hydrophobic phase. There is also a need for polymers they can help in addressing the needs above.

### BRIEF SUMMARY OF THE INVENTION

The present invention addresses at least some of the needs above by providing a linear amphiphilic di-block or tri-block copolymer selected from the group consisting of:

- (block A)-(block B) di-block copolymers,
- (block A)-(block B)-(block A) tri-block copolymers, and
- (block B)-(block A)-(block B) tri-block copolymers,

wherein

- block A is a hydrophilic block, and
- block B is a hydrophobic block,

wherein:

- block A comprises units deriving from vinyl-pyrrolidone or acrylamide, and
- block B comprises units deriving from a linear or branched alkyl acrylate or methacrylate, wherein the alkyl group comprises at least 5 carbon atoms, preferably at least 6 carbon atoms.

The invention also relates to a practical process of making the block copolymer above.

The invention also relates to the use in an emulsion (or to the emulsion itself), of a linear amphiphilic di-block or tri-block copolymer selected from the group consisting of:

- (block A)-(block B) di-block copolymers,
- (block A)-(block B)-(block A) tri-block copolymers, and
- (block B)-(block A)-(block B) tri-block copolymers,

wherein

- block A is a hydrophilic block, and
- block B is a hydrophobic block,

wherein:

- block A comprises units deriving from vinyl-pyrrolidone, and/or
- block B comprises units deriving from a linear or branched alkyl acrylate or methacrylate, wherein the alkyl group comprises at least 5 carbon atoms, preferably at least 6 carbon atoms,

or

- block A comprises units deriving from acrylamide, and
- block B comprises units deriving from a linear or branched alkyl acrylate or methacrylate, wherein the alkyl group comprises at least 5 carbon atoms, preferably at least 6 carbon atoms.

The invention allows especially controlling the stability of emulsions, particularly of simple water-in-oil emulsions, and to stabilize these emulsions. The invention also allows controlling the droplet size of an emulsion, particularly of simple water-in-oil emulsions.

By controlling the droplets size of an emulsion, it is meant that it is possible to obtain an emulsion. The use of the block copolymer, alone or in a mixture with a further emulsifier, allows emulsifying. It is indeed an emulsifier.

By controlling the stability of an emulsion, it is meant that:

- the emulsion remains stable longer with the block copolymer than without the block copolymer, for the same amount of surfactant in the emulsion, and/or
- the emulsion with the block copolymer remains stable as long as, or longer than, an emulsion without the block copolymer and comprising at least the same amount of surfactant than the amount of surfactant together with block copolymer, and/or
- the emulsion with the block copolymer remains stable longer and/or at a higher temperature than with another emulsifying system such as another polymer and/or surfactant that does not comprise the block copolymer, and that comprise at least the same amount of surfactant and/or other polymer than the amount of surfactant together with block copolymer.

Whereas increasing the stability of an emulsion without adding some more emulsifier (surfactant, polymer) is useful, lowering the amount of emulsifier (surfactant, polymer) without decreasing the stability is also useful, as it is for example cost-effective and environment friendly.

The invention is an alternative solution to the use of known emulsifiers or emulsifying systems, that provides at least the same properties, and to many extends advantages. These advantages include a better stability at high temperature, and/or a better stability for some hydrophobic phases (nature and/or amount).

## DETAILED DESCRIPTION OF THE INVENTION

### Definitions

In the present specification, the molecular weight of a polymer, a copolymer or a block refers to the weight-average molecular weight of said polymer, copolymer or block. The weight-average molecular weight of the polymer or copolymer can be measured by gel permeation chromatography (GPC) or steric exclusion chromatography (SEC). In the present specification, the molecular weight of a graft, side-chain, core, branch, block or backbone refers to the molecular weight calculated from the amounts of monomers, polymers, initiators and/or transfer agents used to make the said graft, side-chain, core, branch, block or backbone. It is also referred to "theoretical" or "calculated" or "targeted" molecular weight. The one skilled in the art knows how to calculate these molecular weights. The ratios by weight between blocks refer to the ratios between the amounts of the compounds used to make said moieties, considering an extensive polymerization.

Typically, the molecular weight M of a block is calculated according to the following formula: $M = \sum_i M_i * \frac{n_i}{n_{precursor}}$, wherein $M_i$ is the molecular weight of a monomer i, $n_i$ is the number of moles of a monomer i, and $n_{precusor}$ is the number of moles of a compound the macromolecular chain of the block will be linked to. Said compound may be a transfer agent or a transfer group, or a previous block. If it is a previous block, the number of moles may be considered as the number of moles of a compound the macromolecular chain of said previous block has been linked to, for example a transfer agent or a transfer group. It may be also obtained by a calculation from a measured value of the molecular weight of said previous block. If two blocks are simultaneously grown from a previous block, at both ends, the molecular weight calculated according to the above formula should be divided by two.

The block copolymer comprises two different blocks, block A, and block B. It is selected from the group consisting of (block A)-(block B) di-block copolymers, (block A)-(block B)-(block A) tri-block copolymers, and (block B)-(block A)-(block B) tri-block copolymers. The block copolymer is a linear block copolymer. By linear it is meant that the blocks arrangement is linear. However, a block may be a block having a comb polymer structure, that is comprising repetitive units comprising a polymeric moiety (macromonomers).

A block is defined by repeating units it comprises. A block may be defined by naming a polymer, or by naming monomers it is derived from. In the present specification, a unit deriving from a monomer is understood as a unit that may be directly obtained from the said monomer by polymerizing. Thus, a unit deriving from an ester of acrylic or methacrylic acid does not encompass a unit of formula $-CH-CH(COOH)-$, $-CH-C(CH_3)(COOH)-$, $-CH-CH(OH)-$, $-CH-C(CH_3)(OH)-$, obtained for example by polymerizing an ester of acrylic or methacrylic acid, or a vinyl acetate, and then hydrolyzing. A unit deriving from acrylic acid or methacrylic acid encompasses for example a unit obtained by polymerizing a monomer (for example an alkyl acrylate or methacylate) and then reacting (for example hydrolyzing) to obtain units of formula $-CH-CH(COOH)-$ or $-CH-C(CH_3)(COOH)-$. A unit deriving from vinyl alcohol encompasses for example a unit obtained by polymerizing a monomer (for example a vinyl ester) and then reacting (for example hydrolyzing) to obtain units of formula $-CH-CH(OH)-$ or $-CH-C(CH_3)(OH)-$.

A block may be a copolymer, comprising several kind of repeating units, deriving form several monomers. Hence, block A and block B are different polymers, deriving from different monomers, but they may comprise some common repeating units (copolymers). Block A and Block B preferably do not comprise more than 50% of a common repeating unit (derived from the same monomer).

Hydrophilic or hydrophobic properties of a block refer to the property said block would have without the other block(s), that is the property of a polymer consisting of the same repeating units than said block, having the same molecular weight. By hydrophilic block, polymer or copolymer, it is meant that the block, polymer or copolymer does not phase separate macroscopically in water at a concentration from 0,01 % and 10% by weight, at a temperature from 20˚C to 30˚C. By hydrophobic block, polymer or copolymer, it is meant that the block, polymer or copolymer does phase separate macroscopically in the same conditions.

<u>Block copolymer</u>

In one embodiment, copolymers that can be used in emulsions, according to the invention, have:

- block A comprising units deriving from vinyl-pyrrolidone (more particularly N-vinyl-pyrrolidone "VP" or "NVP"), and/or
- block B comprising units deriving from a linear or branched alkyl acrylate or methacrylate, wherein the alkyl group comprises at least 5 carbon atoms, preferably at least 6 carbon atoms,

In one embodiment, block copolymers that can be used in emulsions, according to the invention, have:

- block A comprising units deriving from acrylamide, and
- block B comprising units deriving from a linear or branched alkyl acrylate or methacrylate, wherein the alkyl group comprises at least 5 carbon atoms, preferably at least 6 carbon atoms.

In these embodiments block A can comprise units deriving from vinyl-pyrrolidone (more particularly NVP) and units deriving from acrylamide.

Both block A and block B comprise units deriving from mono-alpha-unsaturated monomers. Preferably all the units of block A and block B derive from alpha-unsaturated monomers, preferably mono-alpha-unsaturated monomers.

A particular block copolymer that is particularly useful has:

- block A comprising units deriving from vinyl-pyrrolidone (more particularly NVP) or acrylamide, and
- block B comprising units deriving from a linear or branched alkyl acrylate or methacrylate, wherein the alkyl group comprises at least 5 carbon atoms, preferably at least 6 carbon atoms.

In this particular embodiment block A can comprise units deriving from vinyl-pyrrolidone (more particularly NVP) and units deriving from acrylamide.

The particular block copolymer has an especially suitable effect in emulsions, particularly in water-in-oil emulsions, particularly in water-in-oil emulsions wherein the hydrophobic phase is a hydrocarbon, particularly wherein the hydrocarbon is aliphatic. However the particular block copolymer can be used in other destinations.

Some particular features of the block copolymers are presented below. They can be applied to any of the block copolymers that can be used in emulsions, according to the invention, and/or to the particular block copolymer, when suitable and/or applicable.

The alkyl group of the linear or branched alkyl acrylate or methacrylate has at least 5, preferably at least 6 carbon atoms. It can have for example 8 or 12 carbon atoms. Examples of suitable alkyl groups are n-hexyl, n-octyl, isooctyl, 2-ethylhexyl, and lauryl.

The weight ratio between block(s) B and block(s) A can be of from 1/99 to 99/1, preferably from 40/60 to 95/5, preferably from 80/20 to 95/5. The weight ratio can be adjusted depending on the use of the polymer, for example for use in emulsions, preferably water in oil emulsions, typically to tune the stability and/or the droplet size of the dispersed phase.

It is mentioned that block A can comprise optional units, further to the units deriving from vinyl-pyrrolidone or to the units deriving from acrylamide. These further units can represent up to 90% by weight of block A, preferably up to 50%. These further units, if present can represent at least 2% by weight of block A. If block A comprises more than x% by weight of units deriving from vinyl-pyrrolidone, then units deriving from acrylamide in block A in an amount of less than x% would typically be understood as further units. If block A comprises more than y% by weight of units deriving from acrylamide, then units deriving from vinyl-pyrrolidone in block A in an amount of less than y% would typically be understood as further units. The optional further units in block A can be hydrophilic or hydrophobic, preferably hydrophilic. Their nature and/or amount are such that block A is hydrophilic.

It is mentioned that block B can comprise optional units, further to the units deriving from the linear or branched alkyl acrylate or methacrylate. These further units can represent up to 90% by weight of block B, preferably up to 50%. These further units, if present can represent at least 2% by weight of block B. The optional further units in block B can be hydrophilic or hydrophobic, preferably hydrophobic. Their nature and/or amount are such that block B is hydrophobic.

In one embodiment both block A and block B comprise some optional further units.

In a preferred embodiment:

- block A comprises at least 90% by weight of units deriving from vinyl-pyrrolidone or acrylamide, and/or
- block B comprises at least 90% by weight of units deriving from the linear or branched alkyl acrylate or methacrylate.

Examples of hydrophobic further units that can be comprised in block A and/or block B, preferably in block B, include units deriving from the following monomers:

- methylacrylate, ethylacrylate, n-propylacrylate, n-butylacrylate, methylmethacrylate, ethylmethacrylate, n-propylmethacrylate, n-butylmethacrylate,
- vinyl Versatate,
- vinyl acetate,
- vinylamine amides,
- acrylonitrile, and
- vinylaromatic compounds such as styrene.

Examples of hydrophilic further units that can be comprised in block A and/or block B, preferably in block A, include units deriving from the following monomers:

- vinyl alcohol,
- acrylamide, methacrylamide,
- vinyl-pyrrolidone,
- alpha-ethylenically-unsaturated, preferably mono-alpha-ethylenically-unsaturated, monocarboxylic acids, such as acrylic acid, methacrylic acid,
- monoalkylesters of alpha-ethylenically-unsaturated, preferably mono-alpha-ethylenically-unsaturated, dicarboxylic acids,
- monoalkylamides of alpha-ethylenically-unsaturated, preferably mono-alpha-ethylenically-unsaturated, dicarboxylic acids,
- polyethylene oxide (meth)acrylate (i.e. polyethoxylated (meth)acrylic acid),
- hydroxyalkylesters of alpha-ethylenically-unsaturated, preferably mono-alpha-ethylenically-unsaturated, monocarboxylic acids, such as 2-hydroxyethylacrylate,
- hydroxyalkylamides of alpha-ethylenically-unsaturated, preferably mono-alpha-ethylenically-unsaturated, monocarboxylic acids,
- dimethylaminoethyl (meth)acrylate, dimethylaminopropyl (meth)acrylate, ditertiobutylaminoethyl (meth)acrylate, dimethylaminomethyl (meth)acrylamide, dimethylaminopropyl (meth)acrylamide;
- ethylenimine, vinylamine, 2-vinylpyridine, 4- vinylpyridine;
- alpha-ethylenically-unsaturated, preferably mono-alpha-ethylenically-unsaturated, compounds comprising a sulphonic acid group, and salts of alpha-ethylenically-unsaturated, preferably mono-alpha-ethylenically-unsaturated,

compounds comprising a sulphonic acid group, such as vinyl sulphonic acid, salts of vinyl sulfonic acid, vinylbenzene sulphonic acid, salts of vinylbenzene sulphonic acid, alpha-acrylamidomethylpropanesulphonic acid, salts of alpha-acrylamidomethylpropanesulphonic acid 2-sulphoethyl methacrylate, salts of 2-sulphoethyl methacrylate, acrylamido-2-methylpropanesulphonic acid (AMPS), salts of acrylamido-2-methylpropanesulphonic acid, and styrenesulfonate (SS).

- trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido (also called 2-(acryloxy)ethyltrimethylammonium, TMAEAMS) chloride, trimethylammonium ethyl (meth)acrylate (also called 2-(acryloxy)ethyltrimethylammonium, TMAEAMS) methyl sulphate, trimethyl ammonium propyl (meth)acrylamido chloride, vinylbenzyl trimethyl ammonium chloride,
- diallyldimethyl ammonium chloride,
- monomers having the following formula:

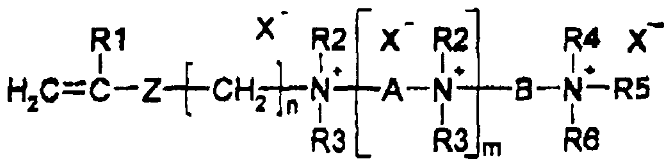

wherein

- $R_1$ is a hydrogen atom or a methyl or ethyl group;
- $R_2$, $R_3$, $R_4$, $R_5$ and $R_6$, which are identical or different, are linear or branched $C_1$-$C_6$, preferably $C_1$-$C_4$, alkyl, hydroxyalkyl or aminoalkyl groups;
- m is an integer from 1 to 10, for example 1;
- n is an integer from 1 to 6, preferably 2 to 4;
- Z represents a -C(O)O- or -C(O)NH- group or an oxygen atom;
- A represents a $(CH_2)_p$ group, p being an integer from 1 to 6, preferably from 2 to 4;
- B represents a linear or branched $C_2$-$C_{12}$, advantageously $C_3$-$C_6$, polymethylene chain optionally interrupted by one or more heteroatoms or heterogroups, in particular O or NH, and optionally substituted by one or more hydroxyl or amino groups, preferably hydroxyl groups;
- X, which are identical or different, represent counterions,

- alpha-ethylenically-unsaturated, preferably mono-alpha-ethylenically-unsaturated, monomers comprising a phosphate or phosphonate group,

While block B is usually a neutral block, block A might be discriminated as regard to its electrical behavior or nature. It means that block A may be a neutral block, or a polyionic block (a polyanionic block, or a polycationic block). It is further mentioned that the electrical behavior or nature (neutral, polyanionic or polycationic) may depend on the pH, typically the pH of the emulsion. By polyionic it is meant that the block comprises ionic (anionic or cationic) repetitive units whatever the pH, or that the block comprises repetitive units that may be neutral or ionic (anionic or cationic) depending on the pH of the emulsion (the units are potentially ionic). A unit that may be neutral or ionic (anionic or cationic), depending on the pH of the composition, will be thereafter referred as an ionic unit (anionic or cationic), or as a unit deriving from an ionic monomer (anionic or cationic), whatever it is in a neutral form or in an ionic form (anionic or cationic). The one skille din the art can discriminate easily neutral units, anionic units, and cationic units in the list of optional further hydrophilic units. Block A is preferably neutral (units deriving from vinyl-pyrrolidone and units deriving from acrylamide are considered as neutral).

It is further mentioned that the block copolymer may be soluble in water, ethanol, and/or in a hydrophobic compound such as a hydrophobic phase of an emulsion for example a hydrocarbon, such as an aromatic hydrocarbon or an aliphatic hydrocarbon. In a preferred embodiment, the block copolymer is soluble in water, in ethanol, in a mixture of water and ethanol, in Diesel Q8 and/or in Exxsol 200.

The block copolymer may be introduced in its destination, for example in the emulsion, or in the mixture of compounds comprised in the emulsion, in a solid form, a dispersed form or in a solution form.

Process for preparing the block copolymer

There are several methods for making block copolymers. Some methods for making such copolymers are provided below.

It is possible for example to use anionic polymerization with sequential addition of 2 monomers as described for example by Schmolka, J. Am. Oil Chem. Soc. 1977, 54, 110; or alternatively Wilczek-Veraet et al., Macromolecules 1996, 29, 4036. Another method which can be used consists in initiating the polymerization of a block polymer at each of the ends of another block polymer as described for example by Katayose and Kataoka, Proc. Intern. Symp. Control. Rel. Bioact. Materials, 1996, 23, 899.

In the context of the present invention, it is recommended to use living or controlled polymerization as defined by Quirk and Lee (Polymer International 27, 359 (1992)). Indeed, this particular method makes it possible to prepare polymers with a narrow dispersity and in which the length and the composition of the blocks are controlled by the stoichiometry and the degree of conversion. In the context of this type of polymerization, there are more particularly recommended the copolymers which can be obtained by any so-called living or controlled polymerization method such as, for example:

- free-radical polymerization controlled by xanthates or trithiocarbonates according to the teaching of Application WO 98/58974 and Patent US 6,153,705,
- free-radical polymerization controlled by dithioesters or trithiocarbonates, according to the teaching of Application WO 98/01478,
- free-radical polymerization controlled by dithioesters according to the teaching of Application WO 99/35178,
- free-radical polymerization controlled by dithiocarbamates according to the teaching of Application WO 99/35177 or WO 99/31144,
- free-polymerization using nitroxide precursors according to the teaching of Application WO 99/03894,
- free-radical polymerization controlled by dithiocarbazates according to the teaching of Application WO 02/26836,
- free-radical polymerization controlled by halogenated Xanthates according to the teaching of Application WO 00/75207,
- free-radical polymerization controlled by dithiophosphoroesters according to the teaching of Application WO 02/10223,
- free-radical polymerization controlled by a transfer agent in the presence of a disulphur compound according to the teaching of Application WO 02/22688,
- atom transfer radical polymerization (ATRP) according to the teaching of Application WO 96/30421,
- free-radical polymerization controlled by iniferters according to the teaching of Otu et al., Makromol. Chem. Rapid. Commun., 3, 127 (1982),
- free-radical polymerization controlled by degenerative transfer of iodine according to the teaching of Tatemoto et al., Jap. 50, 127, 991 (1975), Daikin Kogyo Co Ltd Japan, and Matyjaszewski et al., Macromolecules, 28, 2093 (1995),
- group transfer polymerization according to the teaching of Webster O.W., "Group Transfer Polymerization", p. 580-588, in the "Encyclopedia of Polymer Science and Engineering", Vol. 7, edited by H.F. Mark, N.M. Bikales, C.G. Overberger and G. Menges, Wiley Interscience, New York, 1987,
- radical polymerization controlled by tetraphenylethane derivatives (D. Braun et al., Macromol. Symp., 111, 63 (1996)),
- radical polymerization controlled by organocobalt complexes (Wayland et al., J. Am. Chem. Soc., 116, 7973 (1994)).

Preferred processes are sequenced living free-radical polymerization processes, involving the use of a transfer agent. Preferred transfer agents are agents comprising a transfer group of formula -S-CS-; for example -S-C(S)-Y-, -S-C(S)-S-, or -S-P(S)-Y-, or -S-P(S)-S-, wherein Y is an atom different from sulfur, such as an oxygen atom, a nitrogen atom, a carbon atom, or a silicon atom, these atoms being optionally substituted to complete their valency. The transfer groups include dithioester groups, thioetherthione groups, dithiocarbamate groups, dithiphosphoroesters, dithiocarbazates, trithiocarbonate groups and xanthate groups. Examples of groups comprised in preferred transfer agents include groups of formula $-S-C(S)-NR-NR'_2$, $-S-C(S)-NR-N=CR'_2$, -S-C(S)-O-R, $-S-C(S)-CR=CR'_2$, -S-C(S)-R, -S-C(S)-S-R and -S-C(S)-X, wherein R and R' are or identical or different hydrogen atoms, or organic groups such as hydrocarbyl groups, optionally substituted, optionally comprising heteroatoms, and X is an halogen atom. A preferred polymerization process is a living radical polymerization using xanthates. A preferred xanthate is Rhodixan A1 (O-ethyl S-(1-methoxycarbonyl) ethyl xanthate) of formula: Et-O-CS-S-CH($CH_3$)(COOMe).

Copolymers obtained by a living or controlled free-radical polymerization process may comprise at least one transfer agent group at an end or at the center of the polymer chain. In some particular embodiments such a group is removed or deactivated.

A "living" or "controlled" radical polymerization process that can be used used to make the block copolymers comprises the steps of:

a) reacting a mono-alpha-ethylenically-unsaturated monomer, at least a free radicals source compound, and a transfer agent, to obtain a first block, the transfer agent being bounded to said first block,
b1) reacting the first block, another mono-alpha-ethylenically-unsaturated monomer, and, optionally, at least a radical source compound, to obtain a diblock or triblock copolymer,
b2) optionally, repeating once step b1) to obtain a triblock copolymer, and then
c) optionally, eliminating and/or deactivating the transfer agent or the transfer group thereof, and/or a step of eliminating by-products of the polymerization, elimination and/or deactivation of the transfer group.

If the transfer agent comprises two transfer groups, then a triblock copolymer can be obtained after step b1). If the transfer agent is a trithiocarbonate, preferably a symmetrical one, then a triblock copolymer can be obtained after step b1). If the transfer agent comprises only one transfer group, different from a trithiocarbonate, then a diblock copolymer is obtained after step b1). A triblock copolymer can be then obtained after step b2). In the different steps the one skilled in the art will use the appropriate monomers to obtain the desired compositions of the blocks. These procedures are known by the one skilled in the art.

Step c) can be useful for improving color and/or color of the polymer and/or of a medium comprising the same. It can be useful also to prevent further reaction such as polymerization upon contacting the polymer with reagents, when it's used.

The polymerization can be carried out in an aqueous and/or organic solvent medium. The polymerization can also be carried out in a substantially neat melted form (bulk polymerization), or according to a latex type process in an aqueous medium, or according to a water-in-oil polymerization process.

In a particular process the particular block copolymer is prepared by a process comprising the steps of:

a) polymerizing monomer(s) comprising the linear or branched alkyl acrylate or methacrylate to from at least one block B, then
b) polymerizing monomers comprising vinyl-pyrrolidone, to form at least one block A onto the at least block B.

Preferably:

- step a) comprises contacting an initiator, the monomer(s) comprising the linear or branched alkyl acrylate or methacrylate, and a transfer agent comprising a transfer group of formula -CS-S-, and
- step b) comprises contacting the product of step a) with monomer(s) comprising vinylpyrrolidone, and optionally an initiator.

Preferably the process comprises, after polymerization steps:

- a step of eliminating and/or deactivating the transfer group, and/or
- a step of eliminating by-products of the polymerization, elimination and/or deactivation of the transfer group.

The molecular weight of the block copolymer is preferably comprised between 1000 and 100000 g/mol. It is more preferably comprised between 2000 and 20000 g/mol. Within these ranges, the weight ratio of each block may vary. It is however preferred that each block has a molecular weight above 500 g/mol, and preferably above 1000 g/mol. Within these ranges, the weight ratio between block(s) A and block(s) B (ratio block B / block A) is preferably of from 40/60 to 95/5, and more preferably of from 50/50 to 95/5.

Emulsions and use in emulsions

The invention relates to the use of the block copolymers, preferably the particular block copolymer identified above, in emulsions. The emulsions comprise a dispersed phase in an external phase. In direct emulsions (oil-in-water), preferably simple direct emulsions as opposed to multiple water-in-oil-in-water emulsions, the dispersed phase is a hydrophobic phase, and the external phase is a hydrophilic phase, preferably an aqueous phase. In reverse emulsions (water-in-oil), preferably simple reverse emulsions as opposed to multiple water-in-oil-in-water emulsions, the dispersed phase is a hydrophilic phase, preferably an aqueous phase, and the external phase is a hydrophobic phase.

By using a block copolymer in an emulsion, it is meant that the block copolymer is a compound comprised in the emulsion. It may for example have been added to an emulsion, to the compounds comprised in the emulsion, optionally premixed with some of them, prior to emulsifying, or to a dried emulsion or water, prior to mixing said dried emulsion with water to recover an emulsion.

Emulsions may be prepared in a conventional way, mixing the aqueous phase and the hydrophobic phase, the surfactant, and the block copolymer, providing some energy for emulsifying. Emulsions may be for example prepared

with a homogenizer.

In the emulsion the block copolymer is typically used as an emulsifier or as a co-emulsifier. As it can stabilize and/or control the droplets size of the emulsion, the block copolymer can be a stabilizing emulsifier or co-emulsifier.

Thus, the emulsion can be an emulsion, preferably a simple water-in-oil emulsion, comprising a dispersed phase, preferably an aqueous phase, in a, external phase, preferably a hydrophobic phase, and optionally an emulsifier, different from the block copolymer. The emulsifier different from the block copolymer can be a surfactant.

The hydrophobic phase, preferably the hydrophobic phase of a simple water-in-oil emulsion comprising an aqueous phase dispersed in a hydrophobic phase, optionally comprising an emulsifier different from the block copolymer, can comprise a hydrocarbon compound. In one embodiment the hydrocarbon compound comprises an aliphatic compound.

By hydrocarbon compound it is meant a compound or a mixture of compounds consisting only of carbon atoms and hydrogen atoms. In a hydrocarbon phase comprising a hydrocarbon compound, the hydrocarbon compound encompasses all the constituents of the phase that can be considered as hydrocarbon compounds. In an embodiment the hydrophobic external phase comprises at least 50% by weight, preferably at least 75%, preferably at least 90%, of hydrocarbon compound(s).

The hydrocarbon compound can comprise aromatic hydrocarbon compounds and/or aliphatic hydrocarbon compounds. By aromatic hydrocarbon compound it is meant a compound or a mixture of hydrocarbon compounds comprising at least one aromatic group. By aliphatic hydrocarbon compound it is meant a compound or a mixture of hydrocarbon compounds that do not comprise aromatic group(s).

In a hydrocarbon phase comprising an aliphatic hydrocarbon compound, the aliphatic hydrocarbon compound encompasses all the constituents of the phase that can be considered as aliphatic hydrocarbon compounds. In an embodiment the hydrocarbon compound(s) comprises at least 50% by weight, preferably at least 75%, preferably at least 90%, of aliphatic hydrocarbon compound(s).

In one embodiment:

- the emulsion is a simple water-in-oil emulsion comprising an aqueous phase dispersed in a hydrophobic phase, and optionally an emulsifier, different from the block copolymer, and
- the amount of aqueous phase is of from 10 to 99 weight %, relative to the weight of the hydrophobic phase, block copolymer, optional emulsifier, and aqueous phase.

Preferably:

- the emulsion is a simple water-in-oil emulsion comprising an aqueous phase dispersed in a hydrophobic phase, and optionally an emulsifier, different from the block copolymer, and
- the amount of aqueous phase is of from 50 to 95 weight %.

Preferably:

- the emulsion is a simple water-in-oil emulsion comprising an aqueous phase dispersed in a hydrophobic phase, and optionally an emulsifier, different from the block copolymer, and
- the amount of block copolymer and an optional emulsifier is of from 0.1 to 10 weight % of the amount of aqueous phase.

In one embodiment:

- the emulsion is a simple water-in-oil emulsion comprising an aqueous phase dispersed in a hydrophobic phase, and optionally an emulsifier, different from the block copolymer, and
- the amount of block copolymer and optional emulsifier is of from 0.5 to 5 weight %.

Preferably:

- the emulsion is a simple water-in-oil emulsion comprising an aqueous phase dispersed in a hydrophobic phase, and an emulsifier, different from the block copolymer, and
- the mixture of the block copolymer and the emulsifier has a HLB of lower than or equal to 10.

Aqueous phase

The aqueous phase is based on water, and may comprise some further ingredients, such as active agents or monomers.

In a particular embodiment, the hydrophilic phase is a hydrophilic phase of an explosive emulsion. Such a phase comprises water, an oxygen-donating compound and optionally other water-soluble additives. Examples of oxygen-donating compounds include ammonium nitrate. In such a hydrophilic phase, the water content usually varies in the range 2-30 weight %, preferably in the range 2-30 weight %.

Active agents that may be comprised in aqueous phase include organic or inorganic compounds, as long as they are water-soluble or water-dispersible. They may be solubilized in a hydrophilic solvent that is miscible with water, such as methanol, ethanol, propylene glycol, glycerol. Actives may also be in a solid form, dispersed in the aqueous phase.

Examples of actives in an aqueous phase, that may be used in cosmetics, include compounds having a cosmetic effect, a therapeutic effect, and compounds used for treating hair or skin.

Thus, active agents that may be used include hair and skin conditioning agents, such as polymers comprising quaternary ammonium groups, optionally comprised in heterocycles (quaternium or polyquaternium type compounds), moisturizing agents, fixing (styling) agents, more preferably fixing polymers such as homo-, co-, or terpolymers, for example acrylamide, acrylamide/sodium acrylate, sulfonated polystyrene, cationic polymers, polyvinylpyrrolidone, polyvinyl acetate...

Actives that may be comprised in the aqueous phase also include coloring agents, astringents, that may be used in deodorizing compoisitions, such as aluminum salts, zirconium salts, antibacterial agents, anti-inflammatory agents, anesthetizing agents, solar filter agents such as $TiO_2$, or preferably nanometric $TiO_2$, optionally coated.

Actives comprised in the aqueous phase, that may be used in cosmetics, include $\alpha$- and $\beta$- hydroxyacids, such as citric-acid, lactic acid, glycolic acid, salicylic acid, cicarboxylic acids, preferably unsaturated ones comprising from 9 to 16 carbon atoms, such as azelaic acid, C vitamin and derivatives thereof, particularly phosphate-based or glycosyl-based derivatives, biocidal agents, such as preferably cationic ones (for example Glokill PQ, Rhodoaquat RP50, marketed by Rhodia).

Examples of actives comprised in the aqueous phase, that may be used in food industry, include divalent calcium salts (phosphates, chlorides...), that may be used for cross-linking texturing polymers such as alginates, carraghenans. Sodium bicarbonate may also be used.

Examples of actives comprised in the aqueous phase, that may be used in agrochemicals, include hydrophilic pesticides and pesticides hydrophilic nutritive ingredients.

Examples of actives comprised in aqueous phase, that may be used in oil field, include hydrophilic compounds useful for cementing, drilling, or stimulating oil wells (for example par fracturing). Examples include cross-linking catalysts such as lithium salts, chlorides, acetate. Examples also include compounds that degrade polysaccharides, such as carboxylic acids (for example citric acid), enzymes, and oxidizing agents.

Examples of actives comprised the aqueous phase, that may be used in paper industry, include calcium chloride, and hydrochloric acid.

The aqueous phase may comprise monomers to be polymerized, such as acrylamide and/or AMPS, and/or polymerization products thereof. In some particular embodiments the aqueous phase can cationic monomers, to be reacted with monomers or polymers comprised in the hydrophobic phase.

Hydrophobic phase

The hydrophobic phase is not miscible with the aqueous phase. It is often referred to an oily phase. By "not miscible", it is meant that the ingredient or mixture of ingredients of the hydrophobic phase is not more than 10 weight % soluble in water, at a temperature comprised between 20 ˚C and the emulsion-preparation temperature or emulsion-use temperature.

Suitable hydrophobic phases include:

- organic oils, vegetal oils, mineral oils, waxes, for example used in the field of cosmetics,
- hydrophobic phases comprising a hydrocarbon compound, for example aromatic and/or aliphatic hydrocarbon compounds, preferably an aliphatic compound, as mentioned above,
- saturated or unsaturated fatty acids, saturated or unsaturated fatty acid esters, saturated or unsaturated fatty alcohols,
- industrial lubricants or greases, for examples used to lubricate metal, to work metal, or recovered from metal degreasing,
- silicone oils,
- essential oils, and/or
- agrochemical compounds optionally dissolved in a hydrophobic solvent.

In a particular embodiment, the hydrophobic phase a hydrophobic phase of an explosive emulsion. Examples of such a phase include mineral oils, in particular paraffin mineral oils, naphtalene-based oils, vegetable oils, used oils

or diesel oils.

The hydrophobic phase may comprise some further ingredients, such as active agents.

Examples of actives comprised the hydrophobic phase, that may be used in food industry, include actives used in food industry include mono-, di- and triglycerides, essential oils, aromas, and food compatible coloring agents.

Examples of actives comprised the hydrophobic phase, that may be used in cosmetics, include, fragrances, perfumes, silicone oils, such as dimethicones, lipophilic vitamins such as A vitamin.

Examples of actives comprised the hydrophobic phase, that may be used in paints, include, alkydes resins, epoxy resins, (poly)isocyanates masked or not masked.

Examples of actives comprised the hydrophobic phase, that may be used in paper industry include alkylcetene dimer (AKD), and alkenyl succinic anhydride (ASA).

Examples of actives comprised the hydrophobic phase, that may be used in agrochemicals, include α-cyano-phenoxybenzyl carboxylates, α-cyano-halogenophénoxy-carboxylates, N-methylcarbonates comprising aromatic groups, Aldrin, Azinphos-methyl, Benfluralin, Bifenthrin, Chlorphoxim, Chlorpyrifos, Fluchloralin, Fluroxypyr, Dichlorvos, Malathion, Molinate, Parathion, Permethrin, Profenofos, Propiconazole, Prothiofos, Pyrifenox, Butachlor, Metolachlor, Chlorimephos, Diazinon, Fluazifop-P-butyl, Heptopargil, Mecarbam, Propargite, Prosulfocarb, Bromophos-ethyl, Carbophenothion, and Cyhalothrin.

Examples of actives comprised the hydrophobic phase, that may be used in detergency compositions, include silicone antifoaming agents, fragrances and perfumes.

Examples of actives comprised the hydrophobic phase also include organic solvents or mixtures thereof, such as solvent used for cleaning or stripping such as aromatic oil cuts, terpenic compounds such as D- or L- limonenes, and solvents such as Solvesso®, Exxsol®, Diesel. Solvents also include aliphatic esters such as methyl esters of a mixture of acetic acid, succinic acid, glutaric acid (mixture of Nylon monomer preparation by-products), and chlorinated solvents..

Optional Emulsifier

The emulsion optionally comprises an emulsifier different from the block copolymer. The emulsifier may be a surfactant or a polymer. It is mentionned the the emulsion may comprise no optional emulsifier, less than 4% by weight (based on the total weight of the emulsion), or more than (or equal to) 4% by weight. As the block copolymer has also emulsifying properties, the emulsifier different from the block copolymer may also be referred to as a co-emulsifier. A large variety of emulsifiers or mixture of emulsifiers may be used. That usually depends on the phases and on what the emulsion is used for.

In a preferred embodiment, the mixture of the block copolymer and the emulsifier different from the block copolymer has a HLB of lower than or equal to 10. As the block copolymer usually has a HLB of lower than 10, the emulsifier may have a HLB of higher than 10, or of lower than or equal to 10. Using the block copolymer is thus a way of tuning the HLB of emulsifiers, for example of lowering their HLB. Using the block copolymer in combination with an emulsifier different from the block copolymer may allow the use of said emulsifier in emulsifying or stabilizing emulsions wherein such a result would not have been obtained with said emulsifier alone.

Preferred surfactants, as emulsifiers different from the block copolymer, have a HLB of lower than or equal to 10, and may be selected from the group consisting of sorbitan esters, ethoxylated alcohols, ethoxylated alkyl phenols, and ethoxylated castor oils. Examples of such surfactants include:

- sorbitan trioleate,
- sorbitan tristearate,
- polyoxyethylene sorbitol hexastearate,
- lactylated mono- and diglycerides of fat-forming fatty acids,
- ethylene glycol fatty acid ester,
- mono- and diglycerides of fat-forming fatty acids,
- mono- and di glycerides from the glycerolysis of edible fats,
- propylene glycol fatty acid ester,
- propylene glycol monostearate,
- ethylene glycol fatty acid ester,
- sorbitan sesquioleate,
- polyoxyethylene sorbitol 4.5 oleate,
- glycerol monostearate,
- sorbitan partial fatty esters,
- high-molecular-weight fatty amine blend,
- diethylene glycol fatty acid ester
- polyoxyethylene stearyl ether,

- polyoxyethylene oleyl ether,
- polyoxyethylene sorbitol beeswax dervative,
- polyoxyethylene cetyl ether,
- diethylene glycol monolaurate,
- sorbitan monopalmitate,
- sorbitan monooleate polyoxyethylene ester mixed fatty and resin acids blend,
- polyoxypropylene mannitol dioleate,
- polyoxyethylene sorbitol lanolin derivative,
- sorbitan monolaurate,
- sorbitan monooleate,
- polyoxyethylene sorbitol esters of mixed fatty and resin acids,
- polyoxyethylene fatty acid,
- polyoxyethylene sorbitol oleate,
- polyoxyehtylene sorbitan monostearate,
- polyoxyethylene sorbitol tallow esters,
- polyoxyethylene sorbitol tall oil,
- polyoxyethylene lauryl ether,
- polyoxyethylene sorbitan monooleate.

A series of emulsifiers include polymers sold under the trademarks "Hypermer" or "Arlacel" by ICI, and described in U.S. Pat. Nos 4,504,276, 4,509,950, 4,776,966. Examples of interesting emulsifiers include block or graft copolymers of formula $(A\text{-}COO)_mB$, wherein m is of at least 2, A is a polymeric component having a molecular weight of at least 500 and is the residue of an oil-soluble complex mono-carboxylic acid of formula (I):

$$R\text{-}CO\text{-}[\text{-}O\text{-}CR^1H\text{-}(R^2)_nCO\text{-}]_p\text{-}O\text{-}\ CR^1H\text{-}(R^2)_n\text{-}COOH \qquad (I),$$

wherein R is hydrogen or a monovalent or substituted hydrogen group, $R^1$ is hydrogen or a monovalent $C_1$ to $C_{24}$ hydrocarbon group, $R^2$ is a divalent $C_1$ to $C_{24}$ hydrocarbon group, n is 0 or 1, and p is 0 or an integer of up to 200, and B is a polymeric component having a molecular weight of at least 500 and, in the case where m is 2, is a divalent residue of a water-soluble polyalkylene glycol of the following formula (II):

$$H\text{-}[\text{-}O\text{-}CR^3H\text{-}CH_2\text{-}]_q\text{-}\ CR^3H\text{-}CH_2OH \qquad (II)$$

wherein $R^3$ is hydrogen or a $C_1$ to $C_3$ alkyl group, q is of from 10 to 500, or, in the case where m is greater than 2, is the residue of valency m of a water soluble polyether polyol of the following formula (III):

$$R^4\text{-}\{\text{-}[\text{-}O\text{-}CR^3H\text{-}CH_2\text{-}]_r\text{-}OH\}_m \qquad (III)$$

wherein $R^3$ and m have their previous significance, r is of from 0 to 500, provided that de total number of $\text{-}O\text{-}CR^3H\text{-}CH_2\text{-}$ units in the molecule is at least 10, and $R^4$ is the residue of an organic compound containing in the molecule m hydrogen atoms reactive with an alkylene oxide.

Additional emulsifiers include optionally modified polyak(en)yl succinic anhydrides, such as polyisobutene succinic anhydrides. These emulsifiers include for example the reaction product of a polyak(en)yl succinic anhydride with a polar compound comprising in the molecule at least one hydroxyl or amino group. The preferred polyak(en)yl succinic anhydride are poly (isobutenyl) succinic anhydrides having a molecular weight in the range 400 to 5000. The preferred polar compound with which the anhydride is reacted may be a polyol such as ethylene glycol, propylene glycol, glycerol, trimethylol propane, pentaerythritol or sobitol; or with a polyamine, for example ethylene diamine, trimethylene diamine, hexamethylene diamine, dimethylaminopropylamine or diethylaminopropylamine or with a hydroxyamine for example monoethanolamine, diethanolamine, dipropanoamine; tris(hydroxymathyl)aminomethane or dimethylaminoethanol.

If an emulsifier different from the block copolymer is used, the weight ratio between the amount of the block copolymer and the amount of the emulsifier together with the block copolymer may vary. This is usually a matter of cost, performance and environment impact. Thus, for emulsions comprising an emulsifier different from the block copolymer, the weight ratio between the amount of the block copolymer and the amount of the emulsifier together with the block copolymer is typically of from 1% to 50%, being preferably of 5% to 50%, for example of from about 10%.

The amount of aqueous phase is usually of from 10 to 99 weight %, relative to the weight of the hydrophobic phase, block copolymer, optional emulsifier, and aqueous phase. It is preferably of from 50 to 95 weight %.

The amount of block copolymer and optional emulsifier is usually of from 0.1 to 10 weight % of the amount of

aqueous phase. It is preferably of from 0.5 to 5 weight %.

The emulsion may be prepared by any process known by the one skilled in the art. Usually a process for preparing the emulsion comprises the steps of introducing in a recipient the compounds the emulsion comprises (water, hydrophobic phase compound, block copolymer and optional further emulsifier), and mixing with adding energy in the system (vigorous mixing), for example with a homogenizer. In an embodiment the block copolymer is added to the hydrophobic phase prior to mixing with adding energy. The block copolymer may be introduced in several forms: solid, solution, premix with another compound.... In another embodiment the block copolymer is added into an emulsion which has already been prepared.

Particular applications

The emulsion wherein the block copolymer is used can be, for example:

- an emulsion is an explosive emulsion, the hydrophilic phase comprising oxygen-donating compounds,
- an emulsion is a diesel water in oil emulsion wherein the hydrophobic phase comprises diesel, for examples emulsions comprised in "green diesel" formulations,
- an emulsion is a polymerization emulsion comprising a dispersed phase having monomers and/or a polymerization product thereof, and an external phase, or
- a water-in-oil emulsions of a drilling fluid in oilfield, also referred to as drilling mud
- a water-in-oil emulsion of a fracturing fluids in oilfield, for example as disclosed in U.S. Pat. No. 5,633,220.
- an emulsion is an emulsion in a cosmetic products such as creams and milks, for example sunscreens.

The polymerization emulsion can be a simple water-in-oil emulsion comprising an aqueous phase dispersed in a hydrophobic phase, and optionally, an emulsifier, different from the block copolymer, wherein the aqueous phase comprises monomers and/or a polymerization product thereof, for example acrylamide or AMPS and/or polymerization products thereof or therewith. Water-in oil emulsions polymerization also include polymerization of acrylamide-based copolymers for example poly(acrylamide-cationic monomers) copolymers. In water-in-oil polymerization of acrylamide-based polymers or copolymers, block copolymers wherein block A is a polycationic block are sometime preferred.

The following examples illustrate some details or advantages of the invention.

## EXAMPLES

### Example 1 - Block copolymers

The diblock copolymers (BC) listed on table 1 below are prepared, with using a Xanthate transfer agent.

Table 1

| | Block B | Block A |
|---|---|---|
| BC1 (comparative) | Butyl acrylate, 4300 g/mole | Acrylic acid, 2000 g/mole |
| BC2 (comparative) | 2-Ethyl hexyl acrylate, 8000 g/mole | Acrylic acid, 2000 g/mole |
| BC3 | 2-Ethyl hexyl acrylate, 9000 g/mole | Vinyl pyrrolidone, 1000 g/mole |
| BC4 | Isooctyl acrylate, 9000 g/mole | Vinyl pyrrolidone, 1000 g/mole |
| BC5 | Isooctyl acrylate, 13500 g/mole | Vinyl pyrrolidone, 1500 g/mole |

**Preparation of block copolymer BC2: $p(EHA)_{8000}$-b-$p(AA)_{2000}$**

A) Stage 1: Synthesis of the $p(2\text{-}EHA)_{8000}$ monoblock

Composition of the reaction mixture:

- Ethanol: 52.00 g
- 2-ethylhexyl acrylate (2-EHA): 50.00 g
- O-ethyl S-(1-methoxycarbonyl)ethyl xanthate: 1.30 g
- AIBN (azobisisobutyronitrile): 0.513 g

The ethanol, the 2-ethylhexyl acrylate, the azobisisobutyronitrile (AIBN) and the S-ethylpropionyl O-ethyl dithiocarbonate are introduced into a 250 ml round-bottomed flask equipped with a reflux condenser and a magnetic stirrer.
The reaction medium is brought to 70˚C and is maintained at this temperature for 6 hours.
Samples of polymers are regularly withdrawn for monitoring the conversion.
The solids content is 50% at the end of the reaction.

B) Stage 2: Synthesis of the $p(2\text{-EHA})_{8000}$-b- $p(AA)_{2000}$ diblock copolymer

Composition of the reaction mixture:

- Ethanol: 39.00 g
- Acrylic acid (AA): 12.00 g
- AIBN (azobisisobutyronitrile): 0.492 g

The above ingredients are charged to a dry receptacle under a dry nitrogen atmosphere for 20 minutes and are then transferred into the polymerization reactor, comprising 48.0 g of the dried polymer resulting from the preceding stage, using a double-tip syringe.
At the end of the transfer, the reaction mixture is subsequently heated at 70˚C and is maintained at this temperature for 6 hours.
Samples of polymers are withdrawn from time to time to monitor the conversion. The dry matter content is 40% at the end of the reaction.
The reaction mixture is allowed to cool and the solvents are removed using a rotary evaporator.
The solids content is 40% at the end of the reaction.
The molar mass of the copolymer is 10000 g/mol (theoretical value).

**Preparation of block copolymer BC3: $p(EHA)_{9000}$-b-$p(VP)_{1000}$**

A) Stage 1: Synthesis of the $p(2\text{-EHA})_{9000}$ monoblock

Composition of the reaction mixture:

- Tetrahydrofuran: 52.0 g
- 2-ethylhexyl acrylate (2-EHA): 50.0 g
- AIBN (azobisisobutyronitrile): 0.456 g
- O-ethyl S-(1-methoxycarbonyl)ethyl xanthate: 1.16 g

The abovementioned ingredients are introduced into a 250 ml round-bottomed flask equipped with a reflux condenser and a magnetic stirrer.
The reaction medium is brought to 70˚C and is maintained at this temperature for 6 hours.
A sample is then withdrawn and analyzed by steric exclusion chromatography and the number-average molar mass is of the order of 8600 g/mol.

B) Stage 2: Synthesis of the $p(2\text{-EHA})_{9000}$-b- $p(VP)_{1000}$ diblock copolymer

Composition of the reaction mixture:

- Ethanol: 29.0 g
- N-Vinylpyrrolidone (VP): 5.33 g
- AIBN: 0.437 g
- P(2-EHA) of stage 1: 48.0 g

The ethanol, the N-vinylpyrrolidone and the AIBN are introduced together with the dried polymer synthesized during stage 1.
The reaction medium is brought to 70˚C and is maintained at this temperature for 6 hours.
At the end of the reaction, the ethanol is evaporated under vacuum.
The molar mass of the copolymer is 10000 g/mol (theoretical value).

**Preparation of block copolymer BC4: $p(iOA)_{9000}$-b-$p(VP)_{1000}$**

A) Stage 1: Synthesis of the $p(iOA)_{9000}$ monoblock

Composition of the reaction mixture:

- Tetrahydrofuran: 52.0 g
- Isooctyl acrylate (iOA): 50.0 g
- AIBN (azobisisobutyronitrile): 0.456 g
- O-ethyl S-(1-methoxycarbonyl)ethyl xanthate: 1.16 g

The abovementioned ingredients are introduced into a 250 ml round-bottomed flask equipped with a reflux condenser and a magnetic stirrer.

The reaction medium is brought to 70°C and is maintained at this temperature for 6 hours.

A sample is then withdrawn and analyzed by steric exclusion chromatography and the number-average molar mass is of the order of 8400 g/mol.

B) Stage 2: Synthesis of the $p(iOA)_{9000}$-b- p $(VP)_{1000}$ diblock copolymer

Composition of the reaction mixture:

- Ethanol: 29.0 g
- N-Vinylpyrrolidone (VP): 5.33 g
- AIBN: 0.437 g
- P(iOA) of stage 1: 48.0 g

The ethanol, the N-vinylpyrrolidone and the AIBN are introduced together with the dried polymer synthesized during stage 1.

The reaction medium is brought to 70°C and is maintained at this temperature for 6 hours.

At the end of the reaction, the ethanol is evaporated under vacuum.

The molar mass of the copolymer is 10000 g/mol (theoretical value).

**Preparation of block copolymer BC5: $p(iOA)_{13500}$-b-$p(VP)_{1500}$**

The synthesis procedure of this copolymer is based on three distinct steps process. A first stage which is obtaining a block poly(isooctyl acrylate), a second stage which is the synthesis of a block poly(vinyl pyrrolidone) following the first block and a third stage is the oxidation of the xanthate functionality being present at the polymer chain end. This third stage is run with aim to oxidize the xanthate polymer end-groups, thus to make the polymer chemically inert. Such xanthate group might still acts as a transfer agent when, for example, the diblock copolymer is used to stabilize an emulsion during a polymerization process. Steric exclusion chromatography (SEC) was applied to follow the disappearance of the xanthate groups present at the polymer chain-ends upon reaction with hydrogen peroxide. To estimate the percentage of xanthate oxidation, both the RI and the UV chromatograms are used:

- RI chromatogram: the surface of the peaks attributed to the polymer ($A_R$) is directly proportional to the quantity of polymer.
- UV chromatogram: the detection is made at 290 nm, wavelength characteristic of the C=S group of the xanthate (-S-C(=S)-OEt) : the surface of the peaks attributed to the polymer ($A_{UV}$) is directly proportional to the concentration of xanthate groups. Thiocarbonate (-S-C(=O)-OEt) and sulphonate $-SO_3^{\ominus}$ end-groups that might form via oxidation of xanthate end-groups do not absorb at this wavelength.

The RI and UV peaks are overlapped: the xanthate group is chemically bonded to the polymer chains. The percentage of xanthate oxidation is calculated knowing the surface of the UV peaks and the surface of the RI peaks:

| Peak surface | RI | UV | UV adjusted | % of oxidized xanthate polymer end-groups |
|---|---|---|---|---|
| Sample 1 (before treatment with $H_2O_2$) | $A_{R1}$ | $A_{UV1}$ | | 0 |

(continued)

| Peak surface | RI | UV | UV adjusted | % of oxidized xanthate polymer end-groups |
|---|---|---|---|---|
| Sample 2 (after treatment with $H_2O_2$) | $A_{R2}$ | $A_{UV2}$ | $A'_{UV2} = A_{UV2} * \frac{A_{R1}}{A_{R2}}$ | $(A_{UV1}-A'_{UV2}) * 100/A_{UV1}$ |

The SEC of polymers are achieved in THF :

- solvent : THF (flow rate : 1 ml $mn^{-1}$)
- flow marker: toluene
- columns : Phenogel columns (Guard, linear, 1000 Å and 100 Å)
- double detection : Varian differential refractive index detection (RI) and a UV/VIS absorption detector
- calibration with linear PS standards.

The synthesis of the copolymer is carried out in a 1.5 litres glass reactor. The temperature of the reaction mixture is controlled by a cryostat of the Huber type. The mechanical stirring is used with rotation speed of ~200 tr/min. The reactor is also equipped with a reflux (refrigerants with serpentine) sufficiently effective to allow the backward flow of the monomers without losses of product.

Stage 1: Preparation of a poly(isooctyl acrylate) (first block) of theoretical molecular mass of Mn=13500 g/mol

The reactor is purged with Nitrogen at ambient temperature for about 5 minutes. Then the mixture of 276g of Exxsol D100S with 30.4g of isooctyl acrylate and 4.69g of Rhodixan A1 (O-ethyl S-(1-methoxycarbonyl)ethyl xanthate) are introduced as an initial charge under nitrogen flow.
Once reactor is charged the stirring system is turned on and the reaction mixture is heated to 75˚C.
The nitrogen purge is stopped when the reaction temperature is about 70˚C.
The mixture composed of 0.548g of AMBN (2,2'-Azobis-(2-méthylbutyronitryle) and 3.1 g of ethanol is added in one shot to the reactor at 70˚C. This moment marks a time T0.
At time T0 plus 30 minutes, 274g of isooctyl acrylate is added in the continuous way over 120minutes.
At time T0 plus 75 minutes, the initiator solution (1.10g of AMBN dissolved in 6.2g of ethanol) is added continuously over 90 minutes.
Once the feed of the initiator is completed the reaction temperature is increased to 85˚C. Then the heating is continued until time T0 plus 300 minutes.
A sample (~5g) then is taken from the reactor and analyzed by steric exclusion chromatography (SEC) in THF. The solid content is determined by weight analysis and the residual monomer and Rhodixan A1 are checked by High Performances Liquid Chromatography (HPLC). These analytical results are regrouped in the table below.

| isooctyl acrylate | Rhodixan A1 | solid content | Mw | Mn | Mw/Mn |
|---|---|---|---|---|---|
| 0,74% | < 10 ppm | 56,6% | 14300 | 9500 | 1,5 |

Stage 2: First block extension with the second block of poly(vinyl pyrrolidone) of theoretical molecular mass Mn=1500 g/mol to obtain a dibloc copolymer poly(isooctyl acrylate-block-poly(vinyl pyrrolidone)

At time T0 plus ~300 minutes, 33.8g of vinyl pyrrolidone is added in continuous way for 30 minutes to the polymer solution obtained during first stage, keeping reaction temperature at 85˚C.
At time T0 plus 315 minutes, the initiator solution (1.10g of AMBN dissolved in 6.2g of ethanol) is added continuously over 90 minutes.
After complete addition of the various ingredients, the obtained copolymer solution is maintained at 85˚C during about three hours until time T0 plus 600 minutes.
Then the reaction mixture is cooled to 60˚C during one hour.
A sample (~5g) then is taken from the reactor and analyzed by steric exclusion chromatography (SEC) in THF relative to polystyrene standards. The residual monomers and Rhodixan A1 are checked by High Performances Liquid Chromatography (HPLC).

These analytical results are regrouped in the table below.

| isooctyl acrylate | Rhodixan A1 | N-vinyl pyrrolidone | Mw | Mn | Mw/Mn |
|---|---|---|---|---|---|
| < 20 ppm | < 10 ppm | 0,41 % | 14000 | 8900 | 1,6 |

Stage 3: Oxidation of the xanthate functionality being incorporated to the polymer chain end

Once the reaction temperature was decreased to 60˚C, the mixture of 2.84g of sodium hydrogen carbonate ($NaHCO_3$) and 1.79g of sodium carbonate ($Na_2CO_3$) dissolved in 47.8g water is added continuously to the reactor over 30 minutes. The reaction mixture forms an inverse emulsion-like system (water in Exxsol). Then 10.4g of hydrogen peroxide is continuously introduced over 1 hour.

After complete addition of the various ingredients, the obtained copolymer solution is maintained at 60˚C during next two hours. Then reaction mixture is cooled to ambient temperature.

A final product is analysed by steric exclusion chromatography (SEC) in THF. The solid content is determined by weight analysis and the residual monomer and Rhodixan A1 are checked by High Performances Liquid Chromatography (HPLC). These analytical results are regrouped in the table below.

| isooctyl acrylate | Rhodixan A1 | N-vinyl pyrrolidone | UV (290nm) surface decrease | solid content | viscosity at 20˚C (cp) |
|---|---|---|---|---|---|
| < 20 ppm | < 5 ppm | 0,27% | 82% | 50,4 | ~4000 |

## Example 2 - Water in oil emulsions in methyl ester of rapeseed oil

Ingredients:

- Block copolymers BC1 (see table 1).
- Emulsifier: Surfactant Alkamuls OR10 marketed by Rhodia: ethoxylated castor oil of low HLB.
- Hydrophobic phase: Phytorob 926-65, a product marketed by Novance: a methyl ester of rape seed oil (polar oil)
- Aqueous phase: 0.1M NaCl solution in deionized water.

Emulsification procedure:

A solution comprising the block copolymer and/or the emulsifier, is prepared by dissolving the required amounts in the hydrophobic phase. The aqueous phase is added to the preceding solution to a defined ratio aqueous phase / hydrophobic phase. The sample is then mixed with an ultra-turrax apparatus during 2 min at 10000 rpm.

Tests:

Stability is evaluated by following the coalescence as a function of time: coalescence level is expressed as the ratio of:

- the water amount appearing as a separate phase at the bottom of the sample, and
- the total amount of water initially introduced into the sample.

Results are shown on table 2 below

Table 2

| | Reference | BC1 alone | BC1 + emulsifier |
|---|---:|---:|---:|
| dibloc | - | BC1 at 0.5% | BC1 at 0.2% |
| hydrophobic phase | Phytorob 926-65 | Phytorob 926-65 | Phytorob 926-65 |

(continued)

| | Reference | BC1 alone | BC1 + emulsifier |
|---|---|---|---|
| ratio of phases (w/o) | 20/80 | 20/80 | 20/80 |
| additional emulsifier | Alkamuls R10 5% | 0 % | Alkamuls R10 2% |
| Coalescence level after 1 week at 20°C | 100% | 0% | 0% |

**Example 3 - Solubility in an aliphatic hydrocarbon: aliphatic cut of a mineral oil**

Hydrophobic phase: aliphatic cut of a mineral oil: Exxsol D100S, Exxon Mobile

The preparation of a reverse (water in oil) emulsion using the copolyme BC1 in this aliphatic cut of a mineral oil is not possible since, in the above hydrophobic phase, the copolymer bloc BC1 is not soluble.

**Example 4 - Water in oil emulsions in aliphatic cut of a mineral oil**

Ingredients:

- Block copolymers listed in Table 1.
- Emulsifier: Surfactant Alkamuls S80 marketed by Rhodia
- Hydrophobic phase, aliphatic cut of a mineral oil: Exxsol D100S, Exxon Mobile
- Aqueous phase: 0.1 M NaCl solution in deionized water.

Emulsification procedure:

A solution comprising the block copolymer, and optionally the emulsifier, is prepared by dissolving the required amounts in the hydrophobic phase. The aqueous phase is added to the preceding solution to a defined ratio aqueous phase / hydrophobic phase. The sample is then mixed with an ultra-turrax apparatus during 2 min at 10000 rpm.

Emulsions tests

The quality of the emulsion is evaluated at t0 just after emulsification, after 1 week at 50°C, or after 5 weeks at room temperature.
Results are shown on table 3 below.

Table 3

| Emulsion | Reference | BC2 alone | BC3 alone | BC4 alone |
|---|---|---|---|---|
| dibloc | - | BC2 at 3% | BC3 at 3% | BC4 at 3% |
| hydrophobic phase | Exxsol D100 | Exxsol D100 | Exxsol D100 | Exxsol D100 |
| ratio of phases (w/o) | 70/30 | 70/30 | 70/30 | 70/30 |
| emulsifier (sorbitan ester) | Alkamuls S80 3% | - | - | - |
| appearence of the emulsion at t=0 | 2-10 μm flocculated, viscous | 2-25 μm fluid | 2-5 μm fluid | 2-5 μm fluid |
| Stability after 5 weeks at room temperature | some coalescence, flocculated, viscous | some coalescence | no coalescence, fluid, easily redispersed | no coalescence, fluid, easily redispersed |

| Emulsion | Reference | BC5 alone | BC5 + Emulsifier |
|---|---|---|---|
| dibloc | - | 2% | 0,20% |
| hydrophobic phase | Exxsol D100 | Exxsol D100 | Exxsol D100 |
| ratio of phases (w/o) | 70/30 | 70/30 | 70/30 |
| additional emulsifier | Alkamuls S80 2% | - | 1,70% |
| Stability after 1 week at 50°C | coalesced | no coalescence, fluid and easily redispersed | no coalescence, fluid and easily redispersed |

## Claims

**1.** A linear amphiphilic di-block or tri-block copolymer selected from the group consisting of:

- (block A)-(block B) di-block copolymers,
- (block A)-(block B)-(block A) tri-block copolymers, and
- (block B)-(block A)-(block B) tri-block copolymers,

wherein

- block A is a hydrophilic block, and
- block B is a hydrophobic block,

wherein:

- block A comprises units deriving from vinyl-pyrrolidone or acrylamide, and
- block B comprises units deriving from a linear or branched alkyl acrylate or methacrylate, wherein the alkyl group comprises at least 5 carbon atoms, preferably at least 6 carbon atoms.

**2.** A copolymer according to claim 1 wherein the alkyl group of the linear or branched alkyl acrylate or methacrylate is selected from the group consisting of n-hexyl, n-octyl, isooctyl, 2-ethylhexyl, and lauryl.

**3.** A copolymer according to any of the preceding claims wherein the weight ratio between block B and block A is of from 1/99 to 99/1, preferably from 40/60 to 95/5, preferably from 80/20 to 95/5.

**4.** A copolymer according to any of the preceding claims wherein:

- block A comprises at least 90% by weight of units deriving from vinyl-pyrrolidone or acrylamide, and/or
- block B comprises at least 90% by weight of units deriving from the linear or branched alkyl acrylate or methacrylate.

**5.** A copolymer according to any of the preceding claims, wherein

- block A comprises optional further units, being hydrophilic or hydrophobic units, and/or
- block B comprises optional further units, being hydrophilic or hydrophobic units.

**6.** A copolymer according to claim 5, wherein the optional further hydrophilic units are selected from the group consisting of:

- vinyl alcohol,
- acrylamide,
- vinyl-pyrrolidone,
- 2-hydroxyethylacrylate,
- acrylic acid, methacrylic acid,

- vinyl sulphonic acid, salts of vinyl sulfonic acid, vinylbenzene sulphonic acid, salts of vinylbenzene sulphonic acid, alpha-acrylamidomethylpropanesulphonic acid, salts of alpha-acrylamidomethylpropanesulphonic acid 2-sulphoethyl methacrylate, salts of 2-sulphoethyl methacrylate, acrylamido-2-methylpropanesulphonic acid (AMPS), salts of acrylamido-2-inethylpropanesulphonic acid, and styrenesulfonate (SS).

**7.** A copolymer according to claim 5 or 6, wherein the optional further hydrophobic units are selected from the group consisting of:

- methylacrylate, ethylacrylate, n-propylacrylate, n-butylacrylate, methylmethacrylate, ethylmethacrylate, n-propylmethacrylate, n-butylmethacrylate,
- vinyl Versatate,
- vinyl acetate,
- acrylonitrile, and
- styrene.

**8.** A process of making a block copolymer according to one of the preceding claims comprising the steps of:

a) polymerizing monomer(s) comprising the linear or branched alkyl acrylate or methacrylate to from at least one block B, then
b) polymerizing monomers comprising vinyl-pyrrolidone, to form at least one block A onto the at least block B.

**9.** The process according to claim 8, wherein

step a) comprises contacting an initiator, the monomer(s) comprising the linear or branched alkyl acrylate or methacrylate, and a transfer agent comprising a transfer group of formula -CS-S-, and
step b) comprises contacting the product of step a) with monomer(s) comprising vinylpyrrolidone, and optionally an initiator.

**10.** A process according to any claim 9, optionally comprising after the polymerization steps:

- a step of eliminating and/or deactivating the transfer group, and/or
- a step of eliminating by-products of the polymerization, elimination and/or deactivation of the transfer group.

**11.** Use in an emulsion, of a linear amphiphilic di-block or tri-block copolymer selected from the group consisting of:

- (block A)-(block B) di-block copolymers,
- (block A)-(block B)-(block A) tri-block copolymers, and
- (block B)-(block A)-(block B) tri-block copolymers,

wherein

- block A is a hydrophilic block, and
- block B is a hydrophobic block,

wherein:

- block A comprises units deriving from vinyl-pyrrolidone, and
- block B comprises units deriving from a linear or branched alkyl acrylate or methacrylate, wherein the alkyl group comprises at least 5 carbon atoms, preferably at least 6 carbon atoms,
or
- block A comprises units deriving from acrylamide, and
- block B comprises units deriving from a linear or branched alkyl acrylate or methacrylate, wherein the alkyl group comprises at least 5 carbon atoms, preferably at least 6 carbon atoms.

**12.** Use according to claim 11, wherein the block copolymer is a block copolymer according the any of claims 1 to 7.

**13.** Use according to any of claims 11 to 12, as an emulsifier or as a co-emulsifier.

**14.** Use according to any of claims 11 or 13, wherein the emulsion is a simple water-inoil emulsion.comprising an aqueous phase dispersed in a hydrophobic phase, and optionally an emulsifier, different from the block copolymer.

**15.** Use according to claim 14, wherein the emulsifier different from the block copolymer is a surfactant.

**16.** Use according to any of claims 11 to 15, wherein

- the emulsion is a simple water-in-oil emulsion comprising an aqueous phase dispersed in a hydrophobic phase, and optionally an emulsifier, different from the block copolymer, and
- the hydrophobic phase comprises a hydrocarbon compound.

**17.** Use according to claim 16, wherein the hydrocarbon compound comprises an aliphatic compound.

**18.** Use according to any of claims 11 to 17, wherein:

- the emulsion is a simple water-in-oil emulsion comprising an aqueous phase dispersed in a hydrophobic phase, and optionally an emulsifier, different from the block copolymer, and
- the amount of aqueous phase is of from 10 to 99 weight %, relative to the weight of the hydrophobic phase, block copolymer, optional emulsifier, and aqueous phase.

**19.** Use according to any of claims 11 to 18, wherein:

- the emulsion is a simple water-in-oil emulsion comprising an aqueous phase dispersed in a hydrophobic phase, and optionally an emulsifier, different from the block copolymer, and
- the amount of aqueous phase is of from 50 to 95 weight %.

**20.** Use according to any of claims 11 to 19, wherein:

- the emulsion is a simple water-in-oil emulsion comprising an aqueous phase dispersed in a hydrophobic phase, and optionally an emulsifier, different from the block copolymer, and
- the amount of block copolymer and an optional emulsifier is of from 0.1 to 10 weight % of the amount of aqueous phase.

**21.** Use according to claim 24, wherein:

- the emulsion is a simple water-in-oil emulsion comprising an aqueous phase dispersed in a hydrophobic phase, and optionally an emulsifier, different from the block copolymer, and
- the amount of block copolymer and optional emulsifier is of from 0.5 to 5 weight %.

**22.** Use according to any of claims 11 to 21, wherein:

- the emulsion is a simple water-in-oil emulsion comprising an aqueous phase dispersed in a hydrophobic phase, and an emulsifier, different from the block copolymer, and
- the mixture of the block copolymer and the emulsifier has a HLB of lower than or equal to 10.

**23.** Use according to any of claims 11 to 22, wherein

- the emulsion is an explosive emulsion, the hydrophilic phase comprising oxygen-donating compounds,
- the emulsion is a diesel water in oil emulsion wherein the hydrophobic phase comprises diesel,
- the emulsion is a polymerization emulsion comprising a dispersed phase having monomers and/or a polymerization product thereof, and an external phase, or
- the emulsion is an emulsion in a cosmetic product.

**24.** Use according to claim 23, wherein the polymerization emulsion is a simple water-in-oil emulsion comprising an aqueous phase dispersed in a hydrophobic phase, and optionally an emulsifier, different from the block copolymer, and

- the aqueous phase comprises monomers and/or a polymerization product thereof.

**25.** Use according to claim 24, wherein the monomers comprise acrylamide or AMPS.

**Patentansprüche**

**1.** Lineares amphiphiles Diblock- oder Triblockcopolymer aus der Gruppe bestehend aus:

- (Block A)-(Block B)-Diblockcopolymeren,
- (Block A)-(Block B)-(Block A)-Triblockcopolymeren und
- (Block B)-(Block A)-(Block B)-Triblockcopolymeren,

worin

- Block A ein hydrophiler Block ist und
- Block B ein hydrophober Block ist,

worin:

- Block A Einheiten, die sich von Vinylpyrrolidon oder Acrylamid ableiten, umfaßt und
- Block B Einheiten, die sich von einem linearen oder verzweigten Alkylacrylat oder -methacrylat ableiten, umfaßt, wobei die Alkylgruppe mindestens 5 Kohlenstoffatome und vorzugsweise mindestens 6 Kohlenstoffatome enthält.

**2.** Copolymer nach Anspruch 1, worin die Alkylgruppe des linearen oder verzweigten Alkylacrylats oder -methacrylats aus der Gruppe bestehend aus n-Hexyl, n-Octyl, Isooctyl, 2-Ethylhexyl und Lauryl ausgewählt ist.

**3.** Copolymer nach einem der vorhergehenden Ansprüche, worin das Gewichtsverhältnis zwischen Block B und Block A 1/99 bis 99/1, vorzugsweise 40/80 bis 95/5, vorzugsweise 80/20 bis 95/5, beträgt.

**4.** Copolymer nach einem der vorhergehenden Ansprüche, worin:

- Block A mindestens 90 Gew.-% Einheiten, die sich von Vinylpyrrolidon oder Acrylamid ableiten, umfaßt und/oder
- Block B mindestens 90 Gew.-% Einheiten, die sich von einem linearen oder verzweigten Alkylacrylat oder -methacrylat ableiten, umfaßt.

**5.** Copolymer nach einem der vorhergehenden Ansprüche, worin:

- Block A fakultative weitere Einheiten, die hydrophil oder hydrophob sind, umfaßt und/oder
- Block B fakultative weitere Einheiten, die hydrophil oder hydrophob sind, umfaßt.

**6.** Copolymer nach Anspruch 5, bei dem die fakultativen weiteren hydrophilen Einheiten aus der Gruppe bestehend aus:

- Vinylalkohol,
- Acrylamid,
- Vinylpyrrolidon,
- 2-Hydroxyethylacrylat,
- Acrylsäure, Methacrylsäure,
- Vinylsulfonsäure, Salzen von Vinylsulfonsäure, vinylbenzolsulfonsäure, Salzen von VinyZbenzolsulfonsäure, alpha-Acrylamidomethylpropansulfonsäure, Salzen von alpha-Acrylamidomethylpropansulfonsäure, 2-Sulfoethylmethacrylat, Salzen von 2-Sulfoethylmethacrylat, Acrylamido-2-methylpropansulfonsäure (AMPS), Salzen von Acrylamido-2-methylpropansulfonsäure und Styrolsulfonat (SS) ausgewählt sind.

**7.** Copolymer nach Anspruch 5 oder 6, bei dem die fakultativen weiteren hydrophoben Einheiten aus der Gruppe bestehend aus:

- Methylacrylat, Ethylacrylat, n-Propylacrylat, n-Butylacrylat, Methylmethacrylat, Ethylmethacrylat, n-Propylmethacrylat, n-Butylmethacrylat,

- Vinylversatat,
- Vinylacetat,
- Acrylnitril und
- Styrol

ausgewählt sind.

**8.** Verfahren zur Herstellung eines Blockcopolymers nach einem der vorhergehenden Ansprüche, bei dem man:

a) ein oder mehrere Monomere, die das lineare oder verzweigte Alkylacrylat oder -methacrylat umfassen, zur Bildung mindestens eines Blocks B polymerisiert und dann
b) Monomere, die Vinylpyrrolidon umfassen, zur Bildung mindestens eines Blocks A auf den mindestens einen Block B aufpolymerisiert.

**9.** Verfahren nach Anspruch 8, bei dem man
in Schritt a) einen Initiator, das oder die Monomere, die das lineare oder verzweigte Alkylacrylat oder -methacrylat umfassen, und ein Übertragungsmittel mit einer Übertragungsgruppe der Formel -CS-S- in Berührung bringt und in Schritt b) das Produkt von Schritt a) mit einem oder mehreren Monomeren, die Vinylpyrrolidon umfassen, und gegebenenfalls einem Initiator in Berührung bringt.

**10.** Verfahren nach Anspruch 9, bei dem man nach den Polymerisationsschritten gegebenenfalls:

- die Übertragungsgruppe eliminiert und/oder desaktiviert und/oder
- Nebenprodukte der Polymerisation, Eliminierung und/oder Desaktivierung der Übertragungsgruppe entfernt.

**11.** Verwendung eines linearen amphiphilen Diblockoder Triblockcopolymers aus der Gruppe bestehend aus:

- (Block A)-(Block B)-Diblockcopolymeren,
- (Block A)-(Block B)-(Block A)-Triblockcopolymeren und
- (Block B)-(Block A)-(Block B)-Triblockcopolymeren,

worin

- Block A ein hydrophiler Block ist und
- Block B ein hydrophober Block ist,

worin:

- Block A Einheiten, die sich von Vinylpyrrolidon ableiten, umfaßt und
- Block B Einheiten, die sich von einem linearen oder verzweigten Alkylacrylat oder -methacrylat ableiten, umfaßt, wobei die Alkylgruppe mindestens 5 Kohlenstoffatome und vorzugsweise mindstens 6 Kohlenstoffatome enthält, oder
- Block A Einheiten, die sich von Acrylamid ableiten, umfaßt und
- Block B Einheiten, die sich von einem linearen oder verzweigten Alkylacrylat oder -methacrylat ableiten, umfaßt, wobei die Alkylgruppe mindestens 5 Kohlenstoffatome und vorzugsweise mindestens 6 Kohlenstoffatome enthält, in einer Emulsion.

**12.** Verwendung nach Anspruch 11, bei der es sich bei dem Blockcopolymer um ein Blockcopolymer nach einem der Ansprüche 1 bis 7 handelt.

**13.** Verwendung nach einem der Ansprüche 11 bis 12 als Emulgator oder Coemulgator.

**14.** Verwendung nach einem der Ansprüche 11 oder 13, bei der es sich bei der Emulsion um eine einfache Wasser-in-Öl-Emulsion, die eine in einer hydrophoben Phase dispergierte wäßrige Phase und gegebenenfalls einen von dem Blockcopolymer verschiedenen Emulgator umfaßt, handelt.

**15.** Verwendung nach Anspruch 14, bei der es sich bei dem von dem Blockcopolymer verschiedenen Emulgator um ein Tensid handelt.

**16.** Verwendung nach einem der Ansprüche 11 bis 15, bei der

- es sich bei der Emulsion um eine einfache Wasser-in-Öl-Emulsion, die eine in einer hydrophoben Phase dispergierte wäßrige Phase und gegebenenfalls einen von dem Blockcopolymer verschiedenen Emulgator umfaßt, handelt und
- die hydrophobe Phase eine Kohlenwasserstoffverbindung umfaßt.

**17.** Verwendung nach Anspruch 16, bei der die Kohlenwasserstoffverbindung eine aliphatische Verbindung umfaßt.

**18.** Verwendung nach einem der Ansprüche 11 bis 17, bei der:

- es sich bei der Emulsion um eine einfache Nasser-in-Öl-Emulsion, die eine in einer hydrophoben Phase dispergierte wäßrige Phase und gegebenenfalls einen von dem Blockcopolymer verschiedenen Emulgator umfaßt, handelt und
- die Menge der wäßrigen Phase 10 bis 99 Gew.-%, bezogen auf das Gewicht der hydrophoben Phase, des Blockcopolymers, des fakultativen Emulgators und der wäßrigen Phase, beträgt.

**19.** Verwendung nach einem der Ansprüche 11 bis 18, bei der:

- es sich bei der Emulsion um eine einfache Wasser-in-Öl-Emulsion, die eine in einer hydrophoben Phase dispergierte wäßrige Phase und gegebenenfalls einen von dem Blockcopolymer verschiedenen Emulgator umfaßt, handelt und
- die Menge der wäßrigen Phase 50 bis 95 Gew.-% beträgt.

**20.** Verwendung nach einem der Ansprüche 11 bis 19, bei der:

- es sich bei der Emulsion um eine einfache Wasser-in-Öl-Emulsion, die eine in einer hydrophoben Phase dispergierte wäßrige Phase und gegebenenfalls einen von dem Blockcopolymer verschiedenen Emulgator umfaßt, handelt und
- die Menge des Blockcopolymers und des fakultativen Emulgators 0,1 bis 10 Gew.-% der Menge der wäßrigen Phase beträgt.

**21.** Verwendung nach Anspruch 20, bei der:

- es sich bei der Emulsion um eine einfache Wasser-in-Öl-Emulsion, die eine in einer hydrophoben Phase dispergierte wäßrige Phase und gegebenenfalls einen von dem Blockcopolymer verschiedenen Emulgator umfaßt, handelt und
- die Menge des Blockcopolymers und des fakultativen Emulgators 0,5 bis 5 Gew.-% beträgt.

**22.** Verwendung nach einem der Ansprüche 11 bis 21, bei der:

- es sich bei der Emulsion um eine einfache Wasser-in-Öl-Emulsion, die eine in einer hydrophoben Phase dispergierte wäßrige Phase und gegebenenfalls einen von dem Blockcopolymer verschiedenen Emulgator umfaßt, handelt und
- die Mischung des Blockcopolymers und des Emulgators einen HLB-Wert kleiner gleich 10 aufweist.

**23.** Verwendung nach einem der Ansprüche 11 bis 22, bei der

- es sich bei der Emulsion um eine explosive Emulsion handelt, wobei die hydrophile Phase sauerstoffabgebende Verbindungen umfaßt,
- es sich bei der Emulsion um eine Diesel-Wasser-in-Öl-Emulsion handelt, wobei die hydrophobe Phase Diesel umfaßt,
- es sich bei der Emulsion um eine Polymerisationsemulsion, die eine dispergierte Phase mit Monomeren und/oder einem Polymerisationsprodukt davon und eine externe Phase umfaßt, handelt oder
- es sich bei der Emulsion um eine Emulsion in einem kosemtischen Produkt handelt.

**24.** Verwendung nach Anspruch 23, bei der es sich bei der Polymerisationsemulsion um eine einfache Wasser-in-Öl-Emulsion, die eine in einer hydrophoben Phase dispergierte wäßrige Phase und gegebenenfalls einen von dem

Blockcopolymer verschiedenen Emulgator umfaßt, handelt und

- die wäßrige Phase Monomere und/oder ein Polymerisationsprodukt davon umfaßt.

**25.** Verwendung nach Anspruch 24, bei der die Monomere Acrylamid oder AMPS umfassen.

## Revendications

**1.** Copolymère di-bloc ou tri-bloc linéaire amphiphile choisi parmi le groupe constitué :

- de copolymères di-blocs (bloc A)-(bloc B),
- de copolymères tri-blocs (bloc A)-(bloc B)-(bloc A), et
- de copolymères tri-blocs (bloc B)-(bloc A)-(bloc B),

où

- le bloc A est un bloc hydrophile, et
- le bloc B est un bloc hydrophobe,

où :

- le bloc A comprend des motifs dérivés de la vinylpyrrolidone ou de l'acrylamide, et
- le bloc B comprend des motifs dérivés d'un acrylate

ou d'un méthacrylate d'alkyle linéaire ou ramifié, où le groupe alkyle renferme au moins 5 atomes de carbone, de préférence au moins 6 atomes de carbone.

**2.** Copolymère selon la revendication 1, dans lequel le groupe alkyle de l'acrylate ou du méthacrylate d'alkyle linéaire ou ramifié est choisi parmi le groupe constitué d'un groupe n-hexyle, d'un groupe n-octyle, d'un groupe iso-octyle, d'un groupe 2-éthylhexyle et d'un groupe lauryle.

**3.** Copolymère selon l'une quelconque des revendications précédentes, dans lequel le rapport pondéral entre le bloc B et le bloc A est de 1/99 à 99/1, de préférence de 40/60 à 95/5, de préférence de 80/20 à 95/5.

**4.** Copolymère selon l'une quelconque des revendications précédentes, dans lequel :

- le bloc A comprend au moins 90 % en poids de motifs dérivés de la vinylpyrrolidone ou de l'acrylamide, et/ou
- le bloc B comprend au moins 90 % en poids de motifs dérivés de l'acrylate ou du méthacrylate d'alkyle linéaire ou ramifié.

**5.** Copolymère selon l'une quelconque des revendications précédentes, dans lequel

- le bloc A comprend des motifs supplémentaires éventuels, qui sont des motifs hydrophiles ou hydrophobes, et/ou
- le bloc B comprend des motifs supplémentaires éventuels, qui sont des motifs hydrophiles ou hydrophobes.

**6.** Copolymère selon la revendication 5, dans lequel les motifs hydrophiles supplémentaires éventuels sont choisis parmi le groupe constitué :

- de l'alcool vinylique,
- de l'acrylamide,
- de la vinylpyrrolidone,
- de l'acrylate de 2-hydroxyéthyle,
- de l'acide acrylique, de l'acide méthacrylique,
- de l'acide vinylsulfonique, de sels de l'acide vinylsulfonique, de l'acide vinylbenzènesulfonique, de sels de l'acide vinylbenzènesulfonique, de l'acide alpha-acrylamidométhylpropanesulfonique, de sels de l'acide alpha-acrylamidométhylpropanesulfonique, du méthacrylate de 2-sulfoéthyle, de sels du méthacrylate de 2-sulfoéthyle, de l'acide acrylamido-2-méthylpropanesulfonique (AMPS), de sels de l'acide acrylamido-2-méthylpropanesulfonique, et du styrènesulfonate (SS).

**7.** Copolymère selon la revendication 5 ou 6, dans lequel les motifs hydrophobes supplémentaires éventuels sont choisis parmi le groupe constitué :

- de l'acrylate de méthyle, de l'acrylate d'éthyle, de l'acrylate de n-propyle, de l'acrylate de n-butyle, du méthacrylate de méthyle, du méthacrylate d'éthyle, du méthacrylate de n-propyle, du méthacrylate de n-butyle,
- du Versatate de vinyle,
- de l'acétate de vinyle,
- de l'acrylonitrile, et
- du styrène.

**8.** Procédé de production d'un copolymère bloc selon l'une des revendications précédentes, comprenant les étapes consistant à :

a) polymériser un (des) monomère(s) comprenant l'acrylate ou le méthacrylate d'alkyle linéaire ou ramifié pour former au moins un bloc B, puis à
b) polymériser des monomères comprenant la vinylpyrrolidone, pour former au moins un bloc A sur l'au moins un bloc B.

**9.** Procédé selon la revendication 8, dans lequel
l'étape a) comprend la mise en contact d'un amorceur, du (des) monomère(s) comprenant l'acrylate ou le méthacrylate d'alkyle linéaire ou ramifié, et d'un agent de transfert comprenant un groupe de transfert de formule -CS-S-, et l'étape b) comprend la mise en contact du produit de l'étape a) avec un (des) monomère(s) comprenant la vinylpyrrolidone, et éventuellement un amorceur.

**10.** Procédé selon la revendication 9, comprenant éventuellement après les étapes de polymérisation :

- une étape d'élimination et/ou de désactivation du groupe de transfert, et/ou
- une étape d'élimination de produits secondaires de la polymérisation, de l'élimination et/ou de la désactivation du groupe de transfert.

**11.** Utilisation, dans une émulsion, d'un copolymère di-bloc ou tri-bloc linéaire amphiphile choisi parmi le groupe constitué :

- de copolymères di-blocs (bloc A)-(bloc B),
- de copolymères tri-blocs (bloc A)-(bloc B)-(bloc A), et
- de copolymères tri-blocs (bloc B)-(bloc A)-(bloc B),

où

- le bloc A est un bloc hydrophile, et
- le bloc B est un bloc hydrophobe,

où :

- le bloc A comprend des motifs dérivés de la vinylpyrrolidone, et
- le bloc B comprend des motifs dérivés d'un acrylate

ou d'un méthacrylate d'alkyle linéaire ou ramifié, où le groupe alkyle renferme au moins 5 atomes de carbone, de préférence au moins 6 atomes de carbone,
ou

- le bloc A comprend des motifs dérivés de l'acrylamide, et
- le bloc B comprend des motifs dérivés d'un acrylate

ou d'un méthacrylate d'alkyle linéaire ou ramifié, où le groupe alkyle renferme au moins 5 atomes de carbone, de

préférence au moins 6 atomes de carbone.

**12.** Utilisation selon la revendication 11, dans laquelle le copolymère bloc est un copolymère bloc selon l'une quelconque des revendications 1 à 7.

**13.** Utilisation selon l'une quelconque des revendications 11 à 12, en tant qu'agent émulsifiant ou en tant qu'agent co-émulsifiant.

**14.** Utilisation selon l'une quelconque des revendications 11 ou 13, dans laquelle l'émulsion est une émulsion simple eau/huile, comprenant une phase aqueuse dispersée dans une phase hydrophobe, et éventuellement un agent émulsifiant, différent du copolymère bloc.

**15.** Utilisation selon la revendication 14, dans laquelle l'agent émulsifiant différent du copolymère bloc est un agent tensioactif.

**16.** Utilisation selon l'une quelconque des revendications 11 à 15, dans laquelle

- l'émulsion est une émulsion simple eau/huile comprenant une phase aqueuse dispersée dans une phase hydrophobe, et éventuellement un agent émulsifiant, différent du copolymère bloc, et
- la phase hydrophobe comprend un composé hydrocarboné.

**17.** Utilisation selon la revendication 16, dans laquelle le composé hydrocarboné comprend un composé aliphatique.

**18.** Utilisation selon l'une quelconque des revendications 11 à 17, dans laquelle :

- l'émulsion est une émulsion simple eau/huile comprenant une phase aqueuse dispersée dans une phase hydrophobe, et éventuellement un agent émulsifiant, différent du copolymère bloc, et
- la quantité de phase aqueuse est de 10 à 98 % en poids, par rapport au poids de la phase hydrophobe, du copolymère bloc, de l'agent émulsifiant éventuel et de la phase aqueuse.

**19.** Utilisation selon l'une quelconque des revendications 11 à 18, dans laquelle :

- l'émulsion est une émulsion simple eau/huile comprenant une phase aqueuse dispersée dans une phase hydrophobe, et éventuellement un agent émulsifiant, différent du copolymère bloc, et
- la quantité de phase aqueuse est de 50 à 95 % en poids.

**20.** Utilisation selon l'une quelconque des revendications 11 à 19, dans laquelle :

- l'émulsion est une émulsion simple eau/huile comprenant une phase aqueuse dispersée dans une phase hydrophobe, et éventuellement un agent émulsifiant, différent du copolymère bloc, et
- la quantité de copolymère bloc et d'un agent émulsifiant éventuel est de 0,1 à 10 % en poids de la quantité de phase aqueuse.

**21.** Utilisation selon la revendication 20, dans laquelle :

- l'émulsion est une émulsion simple eau/kzuile comprenant une phase aqueuse dispersée dans une phase hydrophobe, et éventuellement un agent émulsifiant, différent du copolymère bloc, et
- la quantité de copolymère bloc et d'agent émulsifiant éventuel est de 0,5 à 5 % en poids.

**22.** Utilisation selon l'une quelconque des revendications 11 à 21, dans laquelle :

- l'émulsion est une émulsion simple eau/huile comprenant une phase aqueuse dispersée dans une phase hydrophobe, et un agent émulsifiant, différent du copolymère bloc, et
- le mélange du copolymère bloc et de l'agent émulsifiant présente un HLB inférieur ou égal à 10.

**23.** Utilisation selon l'une quelconque des revendications 11 à 22, dans laquelle :

- l'émulsion est une émulsion explosive, la phase hydrophile comprenant des composés donneurs d'oxygène,

- l'émulsion est une émulsion eau/huile de diesel où la phase hydrophobe comprend du diesel,
- l'émulsion est une émulsion de polymérisation comprenant une phase dispersée présentant des monomères et/ou un produit de polymérisation de ceux-ci, et une phase externe, ou
- l'émulsion est une émulsion dans un produit cosmétique.

**24.** Utilisation selon la revendication 23, dans laquelle l'émulsion de polymérisation est une émulsion simple eau/huile comprenant une phase aqueuse dispersée dans une phase hydrophobe, et éventuellement un agent émulsifiant, différent du copolymère bloc, et

- la phase aqueuse comprend des monomères et/ou un produit de polymérisation de ceux-ci.

**25.** Utilisation selon la revendication 24, dans laquelle les monomères comprennent l'acrylamide ou l'AMPS.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03068827 A
- WO 03068848 A
- WO 9858974 A
- US 6153705 A
- WO 9801478 A
- WO 9935178 A
- WO 9935177 A
- WO 9931144 A
- WO 9903894 A
- WO 0226836 A
- WO 0075207 A
- WO 0210223 A
- WO 0222688 A
- WO 9630421 A
- US 4504276 A
- US 4509950 A
- US 4776966 A
- US 5633220 A


### Non-patent literature cited in the description


- **SCHMOLKA.** *J. Am. Oil Chem. Soc.,* 1977, vol. 54, 110
- **WILCZEK-VERAET et al.** *Macromolecules,* 1996, vol. 29, 4036
- **KATAYOSE ; KATAOKA.** *Proc. Intern. Symp. Control. Rel. Bioact. Materials,* 1996, vol. 23, 899
- **QUIRK ; LEE.** *Polymer International,* 1992, vol. 27, 359
- **OTU et al.** *Makromol. Chem. Rapid. Commun,* 1982, vol. 3, 127
- **TATEMOTO et al.** Jap. Daikin Kogyo Co Ltd, 1975, vol. 50, 127
- **MATYJASZEWSKI et al.** *Macromolecules,* 1995, vol. 28, 2093
- Group Transfer Polymerization. **WEBSTER O.W.** Encyclopedia of Polymer Science and Engineering. Wiley Interscience, 1987, vol. 7, 580-588
- **D. BRAUN et al.** *Macromol. Symp,* 1996, vol. 111, 63
- **WAYLAND et al.** *J. Am. Chem. Soc.,* 1994, vol. 116, 7973